# EUROPÄISCHE PATENTSCHRIFT

(11) **EP 3 402 448 B1**
(45) Veröffentlichungstag und Bekanntmachung des Hinweises auf die Patenterteilung: **02.12.2020**
(21) Anmeldenummer: 17700321.7
(22) Anmeldetag: 10.01.2017
(51) Int. Cl.: A61F 7/00

(54) **VERSPRÜHBARE KÜHLENDE ZUSAMMENSETZUNG UND VORRICHTUNG ZUM AUFBRINGEN DIESER ZUSAMMENSETZUNG**
SPRAYABLE COOLING COMPOSITION AND APPARATUS FOR ITS' APPLICATION
COMPOSITION DE REFROIDISSEMENT POUR VAPORISER ET APPAREIL POUR LEUR APPLICATION

(30) Priorität: 12.01.2016 DE 102016100448
(43) Veröffentlichungstag der Anmeldung: 21.11.2018
(73) Patentinhaber: Fraunhofer-Gesellschaft zur Förderung der angewandten Forschung e.V., 80686 München (DE)
(72) Erfinder: BRUZZANO, Stefano, 47051 Duisburg (DE); SENGESPEICK, Andreas, 46047 Oberhausen (DE)
(86) Internationale Anmeldenummer: PCT/EP2017/050391
(87) Internationale Veröffentlichungsnummer: WO 2017/121716

(56) Entgegenhaltungen:
- EP-A1- 0 206 982
- WO-A1-84/04883
- WO-A1-2014/026213

## Beschreibung

Die Anmeldung betrifft eine versprühbare kühlende Zusammensetzung und eine Vorrichtung zum Aufbringen dieser Zusammensetzung, wie in den Ansprüchen definiert. Die Zusammensetzung dient insbesondere zum Kühlen von Gewebe; im Wesentlichen ist hierbei menschliches Gewebe zu nennen. Die Zusammensetzung umfasst ein Treibmittel und ein Kälteträgermaterial.

Im deutschsprachigen Raum gibt es pro Jahr etwa 1,5 Millionen Sportverletzungen, die einer ärztlichen Behandlung bedürfen. Oftmals handelt es sich hierbei um Verstauchungen, Prellungen oder Zerrungen, bei denen als wirksame Erstversorgung eine Kältetherapie, beispielsweise mittels Kompressen, Kühlgelen oder Kältesprays erfolgt. In der Literatur wird angegeben, dass die Kühlwirkung möglichst sanft sein sollte und lang (20 bis 30 Minuten) anhalten sollte.

Eine Kältetherapie wird aber nicht nur bei Verletzungen, sondern auch in der postoperativen Versorgung, der REHA oder in der Zahnmedizin eingesetzt. Ferner werden auch Krankheiten wie Venenentzündungen, die zu einem Anschwellen und Verhärten von Venen und zu einer geröteten Haut führen, mit kühlenden Präparaten zur Linderung der Schmerzen behandelt. Schließlich sind auch anderweitig sanfte und lang anhaltende Kühleffekte "Pre-Cooling" erwünscht, da beispielsweise durch eine Herabsetzung der Körpertemperatur die Leistungsfähigkeit von Sportlern gesteigert werden kann.

Beim Einsatz von Kältesprays für die Kältetherapie wird der Vorteil der flexiblen, unmittelbaren und ohne Einschränkung der Bewegungsfreiheit möglichen Anwendung mit dem Nachteil nur kurzer und zum Teil auch unerwünschter starker Kühleffekte erkauft. Als Kältespray werden hierbei in der Regel in Sprühdosen abgefüllte Flüssiggase genutzt. Beim Aufsprühen verdampft dabei die Flüssigkeit und erzeugt ein Kälteempfinden.

Ein fortgeschritteneres Kältespray wird in der EP 0206982 A1 offenbart. In dieser Schrift wird zur Behandlung von schweren Verbrennungen ein zweistufiges Verfahren vorgestellt, bei dem zunächst eine Wasserschicht auf der zu kühlenden Wunde aufgebracht wird, die dann mittels eines Kältesprays zu einer Eisschicht gefroren wird, die zu einem deutlich längeren Kühleffekt führt. Die Anwendung eines derartigen Kältesprays erfordert allerdings einen komplizierten zweistufigen Prozess, der zudem keine gezielte Dosierung erlaubt.

Der vorliegenden Erfindung liegt daher die Aufgabe zugrunde, eine versprühbare kühlende Zusammensetzung anzugeben, die eine sanfte und lang anhaltende Kühlwirkung bereitstellt und die schnell und unproblematisch auf ein zu kühlendes Gewebe aufgebracht werden kann. Vorzugsweise sollte die Zusammensetzung auch derart dosierbar sein, dass Stärke und Dauer des Kühleffekts über die Dauer des Aufsprühens der Zusammensetzung einstellbar ist.

Diese Aufgaben werden durch die Zusammensetzung und die Vorrichtung zum Aufbringen dieser Zusammensetzung gemäß den unabhängigen Ansprüchen gelöst. Unteransprüche und die Beschreibung lehren vorteilhafte Weiterbildungen.

Eine versprühbare Zusammensetzung zum Kühlen von Gewebe und hier insbesondere von menschlichem Gewebe umfasst eine Dispersion aus einem flüssigen Treibmittel und einem in dem Treibmittel dispergierten Kälteträgermaterial. Das Treibmittel weist dabei einen Siedepunkt im Bereich von -20°C bis +37°C auf; das Kälteträgermaterial besitzt einen Schmelzpunkt im Bereich von -10 bis +30°C und weist als wesentlichen Effekt auf, dass die Kühlwirkung von Gewebe auch darauf zurückzuführen ist, dass spätestens während dem Aufbringen der Zusammensetzung auf das zu kühlende Gewebe der Kälteträger in den festen Aggregatszustand überführt wird, sodass nachfolgend Wärme aus dem Gewebe abgeführt werden kann, um diesen Zustand wieder umzukehren.

Ein wesentlicher Effekt der Zusammensetzung beruht ferner darauf, dass die Aggregatszustandsänderungen des Treibmittels einerseits und des Kälteträgers andererseits aufeinander abgestimmt sind. Die Dispersion, die grundsätzlich als Emulsion vorliegt, wird daher vor Anwendung der Zusammensetzung häufig eine Emulsion sein, da dann die Änderung des Aggregatzustands des Kälteträgermaterials von flüssig auf fest in vorteilhafter Weise genutzt werden kann. Das der Erfindung zugrunde liegende Konzept beruht nämlich insbesondere darauf, dass die durch das verdampfende Treibmittel erzeugte Verdunstungskälte nicht direkt zur Kühlung des Gewebes eingesetzt wird, sondern zunächst auf einen Kälteträger übertragen wird, der seinerseits dann in wesentlich besser dosierbarer Form die Kälte dann langsam an das Gewebe abgibt.

Wenn erfindungsgemäß von einer versprühbaren Zusammensetzung die Rede ist, geht es also im Wesentlichen darum, dass die Zusammensetzung so aus einem Vorratsbehälter auftragbar ist, dass sie flächig auf zu kühlendes Gewebe aufgebracht werden kann, insbesondere mittels handelsüblicher Spraydosen und dergleichen. Durch das Versprühen ist eine gleichmäßige, gezielte und schnelle Aufbringung des Treibmittels möglich, sodass die Verdunstungskälte am zu kühlenden Objekt bereitgestellt werden kann und auch erst dort - da die eingesetzte Dispersion eine Emulsion ist - die Änderung des Aggregatszustands des Kälteträgermaterials in den festen Zustand erfolgt. Gleichzeitig bedeutet versprühbar, dass die Zusammensetzung in einer Form vorliegt, die so homogen ist, dass ein gleichmäßiges Aufbringen der Zusammensetzung mittels handelsüblicher Düsen möglich ist. In der Zusammensetzung sind insbesondere also keine weiteren Komponenten in festem Zustand enthalten, deren Partikelgröße so groß ist, dass ein homogenes Verdüsen nicht mehr oder nur schlecht möglich ist und die insbesondere eine Partikelgröße größer 0,5 mm aufweisen. Im Regelfall wird die versprühbare Zusammensetzung allerdings keine Feststoffe enthalten, wenn man von festem, gegebenenfalls verkapseltem Kälteträgermaterial, insbesondere in Form einer als Suspension vorliegenden Zusammensetzung und eventuell vorhandenen Additiven zur Wärmeleitfähigkeitsverbesserung absieht.

Unter Zusammensetzung "zum Kühlen von Gewebe" wird anmeldungsgemäß nicht nur ein System verstanden, bei dem zu kühlendes Gewebe unmittelbar mit der versprühbaren Zusammensetzung bzw. des nach dem Versprühen gebildeten Gemisch, in Kontakt kommt, sondern auch Anwendungsformen, bei denen das Gewebe nur mittelbar mit der Zusammensetzung in Kontakt kommt. Dies kann beispielsweise der Fall sein, wenn die Zusammensetzung in Verbindung mit anderen medizinischen Mitteln eingesetzt wird, wobei insbesondere Tapes, die mit der erfindungsgemäßen Zusammensetzung gekühlt werden, zu nennen sind.

Unter einem flüssigen Treibmittel wird erfindungsgemäß nicht ausschließlich ein Treibmittel verstanden, das unter Normalbedingungen flüssig ist; vielmehr kann das Treibmittel auch unter Druck in einen Behälter mit der versprühbaren Zusammensetzung eingefüllt sein und dort zwar im Wesentlichen als flüssiges Treibmittel (oft auch als Flüssiggas bezeichnet), zu einem gewissen Anteil aber auch gasförmig vorliegen. In welcher Form das Treibmittel konkret vorliegt hängt hierbei insbesondere von dessen Siedepunkt ab. Wesentlich ist allerdings, dass der in der Zusammensetzung beobachtete Siedepunkt unter Normalbedingungen nicht unterhalb von -20°C liegt. Im Regelfall ist dies zumindest dann realisierbar, wenn ein Treibmittel mit einem Siedepunkt der Reinsubstanz von zumindest -45 °C verwendet wird. Häufig wird allerdings ein Treibmittel verwendet werden, das auch als Reinsubstanz einen Siedepunkt größer - 20 °C aufweist. Ein zu niedriger Siedepunkte des Treibmittels kann - wie auch ein ungünstig gewähltes Kälteträgermaterial (hinsichtlich Schmelzenthalpie, Wärmekapazität und Wärmeleitfähigkeit) und ein ungünstiges Mengenverhältnis von Treibmittel zu Kälteträgermaterial - zu einer Gewebeschädigung des mit der Zusammensetzung behandelten Gewebes und im schlimmsten Fall zu Erfrierungen führen. Als flüssiges Treibmittel kommen daher insbesondere niedrige Alkane und Halogenalkane sowie entsprechende Mischungen in Betracht. Zu nennen sind beispielsweise Chlorethan, Propan, Butan und Tetrafluorethan.

Das Kälteträgermaterial weist einen Schmelzpunkt von -10°C bis +30°C auf, wobei der untere Wert insbesondere dadurch bedingt ist, dass erfindungsgemäß die Kältespitzen beim Verwenden herkömmlicher Kältesprays gerade vermieden werden sollen; der obere Wert ist durch die typischen Oberflächentemperaturen von menschlichen Gewebe bedingt, das insbesondere im Bereich der äußeren Extremitäten häufig nur eine Oberflächentemperatur von +30°C aufweist.

Für physiologisch besonders verträgliche Systeme wird man als untere Schranke für den Schmelzpunkt des Kälteträgermaterials häufig einen Schmelzpunkt von zumindest -2°C oft auch von 0°C oder mehr vorsehen. Als obere Grenze wird man - um die Temperaturdifferenz und damit den Wärmefluss zwischen dem Kälteträgermaterial und der Gewebeoberfläche noch ausreichend hoch zu halten - häufig einen Schmelzpunkt von +20°C wählen.

Im Übrigen sei darauf hingewiesen, dass man den Schmelzpunkt im Regelfall auch so wählen wird, dass er mit dem Siedepunkt des Treibmittels einerseits sowie der Verdampfungsenthalpie des Treibmittels und der Schmelzenthalpie des Kälteträgermaterials korrespondiert und somit das zumindest teilweise Einfrieren der Kälteträgermaterials nach dem Auftragen der Zusammensetzung ermöglicht.

Gemäß einer weiteren Ausführungsform sind Treibmittels und Kälteträgermaterial nicht oder nur schlecht miteinander mischbar, insbesondere wenn das Kälteträgermaterial einen Schmelzpunkt von 0°C oder niedriger aufweist. Hierdurch kann in vorteilhafter Weise eine Gefrierpunktserniedrigung vermieden werden. Nur sehr wenig miteinander mischbar bedeutet demnach im Rahmen der vorliegenden Erfindung, dass bei 20°C weniger als 1 mol.-% Treibmittel im Kälteträgermaterial gelöst vorliegt.

Gemäß einer üblichen Ausführungsform ist die Dispersion aus Treibmittel und Kälteträgermaterial so aufgebaut, dass neben dem Treibmittel und dem Kälteträgermaterial keine weiteren Bestandteile mehr vorliegen, die zur Ausbildung einer Dispersion führen, das heißt dass sämtliche weiteren gegebenenfalls in der Zusammensetzung enthaltenen Bestandteile entweder im Treibmittel oder im Kälteträgermaterial im Wesentlichen vollständig löslich sind. Im Wesentlichen vollständig löslich bedeutet hierbei, dass als Grenzfläche der Bereiche des Kälteträgermaterials innerhalb der Dispersion im Wesentlichen eine Grenzfläche mit dem Treibmittel ausgebildet ist und üblicherweise diese Grenzfläche ausschließlich mit dem Treibmittel ausgebildet ist

Gemäß einer weiteren Ausführungsform weist das Treibmittel eine Verdampfungsenthalpie von mindestens 200 kJ/kg auf, wobei erfindungsgemäß die Verdampfungsenthalpie bei 295 K mittels DSC gemessen wird. Treibmittel mit niedrigeren Verdampfungsenthalpien sind zwar grundsätzlich denkbar, allerdings tritt ein signifikanter Kälteeffekt erst ab 200 kJ/kg auf, sodass man häufig Treibmittel mit Verdampfungsenthalpien > 350 kJ/kg einsetzen wird. Üblicherweise wird man die Treibmittel zudem so wählen, dass sie ein möglichst geringes Treibhauspotenzial aufweisen und dementsprechend nicht halogeniert sind oder - sofern halogenierte Treibmittel eingesetzt werden sollen - diese lediglich in die Mischung mit nicht halogenierten Treibmitteln vorliegen.

Gemäß einer weiteren Ausführungsform wird als Kälteträgermaterial eine Erfindung oder ein Gemisch eingesetzt, das eine Schmelzenthalpie von mindestens 100 kJ/kg aufweist. Mit Kälteträgermaterialien, die eine derartige Schmelzenthalpie aufweisen, kann besonders effektiv die durch das Verdampfen des Treibmittels zu Beginn der Anwendung auftretende Kältespitze abgepuffert werden.

Gemäß einer weiteren Ausführungsform wird das Kälteträgermaterial so ausgewählt, dass es eine Wärmeleitfähigkeit von größer 0,15 W/m*K, häufig größer 0,25 W/m*K besitzt. Ein Kälteträgermaterial mit einer derartigen Wärmeleitfähigkeit ist ebenfalls in vorteilhafter Weise dazu geeignet, die Kältespitze, die beim Auftragen der kühlenden Zusammensetzung durch das Verdunsten des Treibmittels zunächst entsteht, abzupuffern, da die entstehende Kälte besonders schnell durch das Kälteträgermaterial abgeführt werden kann.

Gemäß einer weiteren Ausführungsform wird das Kälteträgermaterial daher ausgewählt aus der Gruppe bestehend aus Wasser, Glyzerin, Polyethylenglycol mit einem Molekulargewicht < 2000 g/mol, Alkane mit einem Molekulargewicht < 500 g/mol, Alkylether mit einem Molekulargewicht kleiner 400 g/mol und größer 200 g/mol als Reinsubstanz sowie Gemischen von zumindest zwei Komponenten, ausgewählt aus der Gruppe bestehend aus Wasser, Glyzerin, Polyethylenglycolen, Alkanen und Alkylethern.. Ferner kann das Kälteträgermaterial einen oder mehrere der genannten Stoffe auch nur enthalten. Als längerkettiges Alkan ist hier insbesondere Tetradekan zu nennen als Dialkylether insbesondere Dioctylether.

Für physiologisch besonders verträgliche Systeme wird man im Regelfall ein hydrophiles Kälteträgermaterial wählen, wobei wässrige Systeme im Regelfall die beste Verträglichkeit besitzen.

Gemäß Erfindung ist daher das Kälteträgermaterial Wasser oder besteht im Wesentlichen aus Wasser. Unter "im Wesentlichen bestehen" wird hierbei insbesondere verstanden, dass der Schmelzpunkt des dann vorliegenden wässrigen Gemischs nicht unter -2°C und nicht über +2°C liegt. Im Regelfall wird der Wasseranteil - unabhängig vom Schmelzpuinkt des wässrigen Gemischs - zumindest 95 Gew.-% betragen. Die Verwendung von Wasser bzw. Eis hat den Vorteil, dass beim Umwandeln des energiereicheren flüssigen Zustands in den energieärmeren festen Zustand die beim Einfrieren abgegebene, sehr hohe Erstarrungswärme genutzt werden kann, die zu einem zusätzlichen Abpuffern des Kältepeaks zu Beginn der Anwendung der erfindungsgemäßen Zusammensetzung verwendet werden kann. Daneben hat Wasser den großen Vorteil, dass es mit fast jeglichem Gewebe eine hervorragende physiologische Verträglichkeit aufweist.

Gemäß einer weiteren Ausführungsform kann das Kälteträgermaterial auch in verkapselter Form vorliegen. Gemäß den vorstehenden Definitionen wird dann durch die Kapsel die Grenzfläche zum Treibmittel ausgebildet. Mittels einer derartigen Ausführungsform können dann in der Kapsel enthaltene Materialien eingesetzt werden, die eine weniger gute physiologische Verträglichkeit aufweisen als beispielsweise Wasser. Zudem ist meist eine leichtere Entfernung des Kälteträgermaterials vom Gewebe möglich, sobald die Anwendung der kühlenden Zusammensetzung beendet ist.

Gemäß einer weiteren Ausführungsform weist die Dispersion eine mittlere Teilchengröße der in der Dispersion vorliegenden Minoritätskomponente (bezogen auf Treibmittel und Kälteträgermaterial und deren Gewichtsanteil) von maximal 100 µm auf. Häufig ist die mittlere Teilchengröße maximal 50 µm. Zu besonders großen Oberflächen gelangt man mit mittleren Teilchengrößen von maximal 10 µm Die mittlere Teilchengröße wird hierbei mittels Laserbeugung nach ISO 13320/1 bestimmt. Durch die Teilchengröße bzw. die Teilchengrößenverteilung der Dispersion kann ein wesentlicher Einfluss auf die Schnelle des Wärme- beziehungsweise Kälteaustauschs zwischen Treibmittel und Kälteträgermaterial genommen werden. Dementsprechend sind zu große mittlere Teilchengrößen weniger geeignet, da die Oberfläche der in der Dispersion vorliegenden Teilchen dann deutlich abnimmt und dementsprechend ein weniger schneller Kälteaustausch stattfindet. Unter Teilchengröße wird hierbei die Partikelgröße in Suspensionen und die Tröpfchengröße in Emulsionen verstanden.

Gemäß einer weiteren Ausführungsform ist die mittlere Teilchengröße der in der Dispersion vorliegenden Minoritätskomponente größer als 0,1 µm, insbesondere größer als 1 µm. Bei kleineren Teilchengrößen ist zwar die Oberfläche nochmals vergrößert, allerdings führen sehr kleine Teilchengrößen zu einem Unterkühlungseffekt, sodass die Kristallisiation des Kälteträgermaterials durch kinetische Hemmung erst bei Temperaturen erfolgt, die deutlich unter dem Schmelzpunkt liegen und das Zusammenspiel von Siedepunkt des Treibmittels und Schmelzpunkt des Kälteträgermaterials unbeabsichtigt verändert wird.

Um die gewählten Teilchengrößen in der erfindungsgemäßen Dispersion realisieren zu können, können der Zusammensetzung Emulgatoren, Dispersionsstabilisatoren und Viskositätsmodifikatoren zugesetzt werden. Als Zusätze kommen hierbei insbesondere gewebeverträgliche Zusätze in Betracht. Zu nennen sind daher vor allem nichtionogene Verbindungen, die in pharmazeutischen Formulierungen Verwendung finden, beispielsweise Emulgatoren wie Cetylalkohol, Cetylstearylalkohol, Oleylalkohol, Stearylalkohol, Glycerolfettsäureester wie Glyceroldistearat, Triglyceroldiisostearate,Propylenglycolfettsäureester, Saccharosefettsäureester, Sorbitanfettsäureester, Poloxamere, Fettalkoholpolyglykolether, Fettsäurepolyglykolester, Propylenglycol-Hydroxystearate, Propylenglycol, Glyceryl fettsäureester, Propylenglycoltrihydroxystearate; Polysorbate sowie Stabilsatoren und Verdickungsmittel wie Guargalactomannan, Hydroxypropylcellulose, Hypromellose und daneben anionische Verbindungen wie Xanthan, Carrageen,

Gemäß einer weiteren Ausführungsform umfasst die versprühbare Zusammensetzung zusätzlich ein Bindemittel, insbesondere ein polymeres Bindemittel, oder besteht aus dem flüssigen Treibmittel, dem Kälteträgermaterial und dem Bindemittel. Das Bindemittel dient dabei dazu, die versprühbare Zusammensetzung auf dem zu kühlenden Gewebe präzise applizieren zu können, das heißt, ganz gezielt und dauerhaft nur in einem bestimmten lokalen Bereich aufzubringen. Das Bindemittel wird dabei so ausgewählt werden, dass es eine möglichst gute physiologische Verträglichkeit aufweist, kann aber grundsätzlich jegliches Bindemittel sein, sofern durch das Bindemittel eine gegenüber einer Zusammensetzung ohne das Bindemittel verbesserte, weil präzisere lokale Applikation möglich ist.

Das Bindemittel hat also den Effekt, dass nach Auftragen der kühlenden Zusammensetzung mit fortschreitender Kühldauer kein "Auseinanderlaufen" des Kälteträgermaterials erfolgen kann. Wird beispielsweise Wasser/Eis als Kälteträgermaterial eingesetzt und ist kein Bindemittel enthalten, so ist ohne weiteres ersichtlich, dass bei zu kühlenden Geweben, die nicht eben sind, beispielsweise bei Armen, Beinen und dergleichen, ein Auseinanderlaufen des Kälteträgermaterials unvermeidbar ist - zumindest dann, wenn ein Schmelzen des Kälteträgermaterials erfolgt ist. Das Bindemittel hat ferner den Effekt, dass dieses die Verdampfungsrate des Treibmittels vermindert und somit durch die verzögerte Freisetzung von Verdunstungskälte Kältespitzen beim initialen Auftrag der kühlenden Zusammensetzung weiter vermindert werden können. Daneben führt die Verminderung der Verdampfungsrate des Treibmittels auch dazu, dass ein Verpuffen des Treibmittels wirkungsvoll verhindert werden kann, dass also der wesentliche Anteil der Verdunstungskälte, die durch das verdampfende Treibmittel erzeugt wird, auch auf das Kälteträgermaterial übertragen werden kann.

Gemäß einer Ausführungsform ist das Bindemittel ein Filmbildner. Als Filmbildner sind insbesondere selbständige Filmbildner geeignet, da dann keine weiteren Hilfsstoffe in der Zusammensetzung enthalten sein müssen. Die Filmbildung erfolgt erfindungsgemäß dabei insbesondere durch Trocknungsvorgänge, da das Bindemittel im Regelfall im Treibmittel gelöst vorliegen wird und das Treibmittel während des Aufbringens auf das zu kühlende Gewebe unweigerlich verdampft. Durch die Filmbildung wird auf dem zu kühlenden Gewebe im Regelfall eine haftende aber flexible Schicht ausgebildet, sodass eine dauerhafte und bis zu einem gewissen Grad gegen mechanische Beschädigung geschützte Schicht der erfindungsgemäßen Zusammensetzung ausgebildet wird. Das Bindemittel hat also auch den Vorteil, dass nicht nur ein präzises Auftragen möglich ist, sondern dass auch dann noch eine Kühlwirkung durch die erfindungsgemäße Zusammensetzung bereitgestellt wird, wenn das Gewebe nach Auftragen sofort wieder "in Bewegung gesetzt wird", wie dies beispielsweise bei Sportlern während Sportereignissen der Fall ist. Insbesondere erfüllt die so eingesetzte Zusammensetzung hinsichtlich des mechanischen Schutzes der zu kühlenden Stelle und hinsichtlich der Flexibilität dieselbe Funktion wie ein Sprühpflaster. Durch Wahl und Menge des Bindemittels kann die erfindungsgemäße Zusammensetzung auf den konkreten Einsatzbereich angepasst werden. Ferner hat ein Filmbildner den Vorteil, dass über Art und Menge des Filmbildners die Verdunstungsgeschwindigkeit des Treibmittels reguliert werden kann und daher Kältespitzen nach Auftragen der Zusammensetzung besser abgepuffert werden können und die Dauer der Kühlwirkung verlängert werden kann.

Der erfindungsgemäß als Bindemittel einsetzbare Filmbildner kann insbesondere ausgewählt werden aus der Gruppe bestehend aus Polyvinylacetat, Polyvinylpyrrolidonen, Polyalkylacrylaten, Nitrocellulose, natürlichem Kautschuk, synthetischem Kautschuk, Polyalkylcyanacrylaten, Polyurethanen, Silikonwachsen, Polysiloxanen, vernetzten Polysacchariden und vernetzten Polysaccharidderivaten, vernetzten Polyacrylaten und vernetzten Polyacrylatcopolymeren, Gelatine, Silikonen und Gemischen der genannten Stoffe untereinander oder im Einzelfall auch Gemische der genannten Stoffe mit anderen als Bindemittel geeigneten Materialien. Die genannten polymeren Verbindungen können auch als Copolymere vorliegen, beispielsweise Copolymere wie Poly(methylacrylat-isobuten-monoisopropylmaleat).

Gemäß einer weiteren Ausführungsform weist das (polymere) Bindemittel eine zahlenmittlere, mittels Membranosmometrie ermittelte Molmasse von 1000 bis 200000 g/mol, insbesondere von 10000 bis 100000 g/mol auf. Bindemittel mit höheren zahlenmittleren Molmassen sind zwar grundsätzlich auch denkbar, erzeugen aber in Lösung im Regelfall eine sehr hohe Viskosität, sodass ein Lösen des Bindemittels im Treibmittel erschwert wird oder nicht mehr vollständig möglich ist. Weist dagegen das polymere Bindemittel eine zu geringe zahlenmittlere Molmasse auf, so tritt keine ausreichende Filmbildung auf.

Gemäß einer weiteren Ausführungsform kann (ergänzend oder alternativ zur vorstehend angegebenen zahlenmittleren Molmasse) das Bindemittel eine Glastemperatur kleiner 40 °C uns insbesondere von -20°C bis +30°C aufweisen. Die Glastemperatur bzw. Glasübergangstemperatur wird hierbei mittels dynamischer thermomechanischer Analyse gemessen. Nur der Vollständigkeit halber sei darauf hingewiesen, dass bei Bindemitteln, die als Gemische vorliegen oder als Blockcopolymere für die erfindungsgemäßen Eigenschaften die Glasübergangstemperatur der (gewichtsbezogenen) Majoritätskomponente maßgeblich ist. Die angegebenen Werte für die Glasübergangstemperatur sind insbesondere dann relevant, wenn eine besonders flexible, ein Bindemittel enthaltende Zusammensetzung eingesetzt werden soll. Unterhalb der Glasübergangstemperatur (Erweichungstemperatur) gehen ganz oder teilweise amorphe Polymere vom hochviskosen oder gummielastischen, flexiblen Zustand in den glasartigen oder hartelastischen, spröden Zustand über..

Gemäß einer weiteren Ausführungsform beträgt der Anteil des Treibmittels in der erfindungsgemäßen Zusammensetzung 20 bis 95 Gew.-% und der Anteil des Kälteträgermaterials 5 bis 80 Gew.-% (diese Werte gelten auch dann, wenn ein Bindemittel enthalten ist). Häufig wird der Anteil des Treibmittels 50 bis 90 Gew.-% betragen und der Anteil des Kälteträgermaterials 10 bis 50 Gew.-% sein; das Kälteträgermaterial ist dann also die Minoritätskomponente. Wesentlich für das Verhältnis von Treibmittel (TRM) und Kälteträgermaterial (KTM) ist insbesondere, dass durch die verwendete Menge an Treibmittel eine vollständige oder - falls dies erwünscht ist - auch nur teilweise Überführung des Kälteträgermaterials in den festen Zustand möglich ist. Angenähert gilt hierfür, dass für eine vollständige Umwandlung folgende Ungleichung gilt: m_{TRM} * ΔH_{v TRM} ≥ m_{KTM} * ΔH_{S KTM}, (wobei ΔHᵥ für die molare Verdampfungsenthalpie und ΔH_{S} für die molare Schmelzenthalpie steht).

Gemäß einer weiteren Ausführungsform ist der Anteil des Bindemittels - sofern die Zusammensetzung ein Bindemittel enthält - 48 Gew.-% oder kleiner, üblicherweise reicht aber bereits eine Menge von 10 Gew.-% und oft sogar von 5 Gew.-%. Um eine ausreichende Wirkung des Bindemittels zu erreichen wird im Regelfall zudem eine Menge von mindestens 1 Gew.-% eingesetzt. Die Menge des Bindemittels ist anmeldungsgemäß insbesondere dadurch bestimmt, wie dick die auf dem Gewebe aufgetragene Schicht auszubilden ist. Es versteht sich von selbst, dass bei zu dicken Schichten die Flexibilität der Schicht mit der kühlenden Zusammensetzung beeinträchtigt ist und insofern eine gewisse Obergrenze sinnvoll ist. Insbesondere in diesem Zusammenhang hat sich ein Bindemittelanteil von maximal 10 Gew.-% als sinnvoll herausgestellt.

Gemäß einer weiteren Ausführungsform kann erfindungsgemäße Zusammensetzung das flüssige Treibmittel, das Kälteträgermaterial, das Bindemittel und ein organisches Lösungsmittel enthalten oder hieraus bestehen. Das Lösungsmittel kann dazu eingesetzt werden, die Löslichkeit des Bindemittels in dem Treibmittel zu verbessern. Damit können auch mit schlechter löslichen Bindemitteln die im vorstehenden Absatz angegebenen Mengen an Bindemittel in die Zusammensetzung eingebracht werden. Als organisches Lösungsmittel ist beispielsweise Isopropanol geeignet.

Wie vorstehend ausgeführt wurde, spielt der Anteil des Kälteträgermaterials eine Rolle für die Ausbildung der in der Dispersion vorliegenden Partikelgrößen. Daneben hat allerdings die Menge des Kälteträgermaterials auch einen Einfluss darauf, wie schnell die Verdunstungskälte abgeführt werden kann und auf welchem primären Temperaturniveau nach vollständiger Verdunstung des Treibmittels das Kälteträgermaterial vorliegt. Eine größere Menge des Kälteträgermaterials kann daher - sofern die Oberfläche der in der Dispersion ausgebildeten Partikel ausreichend groß ist - sinnvoll sein, sofern die Aggregatzustandsänderung des Kälteträgermaterials im Wesentlichen vollständig ausgenützt werden kann, da dann ein verhältnismäßig nah an der Schmelztemperatur liegendes primäres Temperaturniveau in dem Kälteträgermaterial ausgebildet wird. Dies hat wiederum den Vorteil, dass die Kühlwirkung länger anhält, da - zumindest bei lebendem Gewebe - der Körper versucht, die Kälte durch zusätzliche Wärmezufuhr möglichst schnell auszugleichen und diese Wärmezufuhr umso stärker ist, je größer die Temperaturdifferenz zwischen Körper und kühlender Zusammensetzung ist. Entsprechendes gilt in eingeschränktem Maß auch für das das Gewebe umgebende Medium, das heißt die Differenz zwischen Temperatur der kühlenden Zusammensetzung und Umgebungstemperatur.

Die vorstehend angegebenen Komponenten und Gewichtsverhältnisse der Zusammensetzungen gewährleisten im Regelfall, dass bei Auftrag der Zusammensetzung auf menschliche Haut mit einer Gewebeoberflächenausgangstemperatur von 30°C die Zeitspanne t₁, während der die Gewebeoberfläche eine Temperatur von mindestens 5°C unter der der Ausgangstemperatur beträgt, deutlich größer als nach dem Stand der Technik ist. Sie weisen insbesondere eine derartige Kühlwirkung von mindestens 5°C während einer Zeitspanne auf, die um mindestens 50 % länger ist als die für eine Zusammensetzung, die kein Kälteträgermaterial enthält (aber ansonsten die identischen Komponenten in den identischen Verhältnissen enthält). Häufig werden mit den erfindungsgemäßen Zusammensetzungen sogar Kühleffekte erreicht, bei denen diese Zeitspanne zumindest doppelt so groß ist wie in den vergleichbaren Zusammensetzungen ohne das erfindungsgemäße Kälteträgermaterial.

Gemäß einer weiteren Ausführungsform kann die Zusammensetzung zusätzlich zu dem Treibmittel, dem Kälteträgermaterial und ggf. dem Bindemittel hinsichtlich des Kühleffekts noch einen wirkverstärkenden Zusatz enthalten (oder aus diesen drei bzw. vier Komponenten bestehen). Als wirkverstärkende Zusätze sind insbesondere chemische, physikalische oder pharmakologische Zusätze zu nennen. Als chemische Zusätze sind insbesondere Verbindungen relevant, bei denen ein zusätzlicher endothermer Zerfall erfolgt (beispielsweise beim Zerfall von Hydraten), als physikalische Zusätze sind insbesondere Nucleierungsmittel zu nennen (beispielsweise als Lebensmittelzusatz vewendete Phytosterole) und als pharmakologische Zusätze beispielsweise Agonisten des Kälte-Menthol-Rezeptors (beispielsweise Menthol selbst). Während bei den ersteren beiden eine tatsächliche Kühlwirkung erfolgt, ist im letzten Fall eine physiologische Kühlwirkung durch Reizung der entsprechenden Nerven relevant. Im Regelfall werden diese Stoffe so gewählt, dass sie im Treibmittel oder im Kälteträgermaterial löslich sind.

Gemäß einer weiteren Ausführungsform enthält die erfindungsgemäße Zusammensetzung neben Treibmittel, Kälteträgermaterial, ggf. vorliegenden Bindemittel und ggf. wirkverstärkendem Zusatz noch weitere Additive. Hier sind insbesondere Prozess- und Produkthilfsstoffe wie Lösemittel, Emulgatoren, Dispersionsstabilisatoren, Antioxidanzien, Wärmeübertragungsmodifikatoren und Viskositätsmodifikatoren zu nennen. Im Regelfall werden diese Stoffe so gewählt, dass sie entweder im Treibmittel oder im Kälteträgermaterial löslich sind.

Die erfindungsgemäße thermoregulierende Zusammensetzung wird üblicherweise mittels einer handelsüblichen Druckgasflasche oder einer vergleichbaren Vorrichtung aufgebracht werden. Die Zusammensetzung kann hierfür in diese Druckgasflasche abgefüllt werden und in bewährter Weise über eine Düse auf das zu kühlende Gewebe aufgesprüht werden.

Gemäß einer alternativen Ausführungsform wird die Aufgabe der vorliegenden Erfindung aber auch durch eine Vorrichtung gelöst, in der die Dispersion aus dem Kälteträgermaterial und dem gegebenenfalls ein Bindemittel enthaltenden Treibmittel erst in situ ausgebildet wird. Eine derartige Erzeugung in situ kann beispielsweise realisiert werden, wenn das Treibmittel (mit ggf. darin enthaltenen Bindemittel) in einem ersten Vorratsbehälter vorgesehen ist und das Kälteträgermaterial in einem zweiten Vorratsbehälter. Durch Betätigung der Vorrichtung werden dann über gegebenenfalls enthaltene Ventile das Treibmittel und das Kälteträgermaterial in einem eingestellten oder einstellbaren Mischungsverhältnis einer Mischkammer zugeführt, in der die Ausbildung der Dispersion erfolgt und in der gegebenenfalls Mittel zum Vermischen von Treibmittel und Kälteträgermaterial vorhanden sind und wobei aus dieser Mischkammer dann über eine Düse die kühlende Zusammensetzung auf das zu behandelnde Gewebe aufgetragen werden kann.

Die erfindungsgemäße Zusammensetzung stellt erstmalig ein einfach zu applizierendes funktionales Material für ein Kältespray zur Verfügung, insbesondere für die medizinische Erstversorgung (auch postoperativ) und Schmerztherapie. Durch den Zusatz des Kälteträgermaterials erfolgen hierbei eine "sanfte" Temperatureinwirkung sowie eine sofortige und lang anhaltende Wirkung. Insbesondere bei Verwendung einer ein Bindemittel enthaltenden Zusammensetzung bietet diese dem Anwender eine uneingeschränkte Bewegungsfreiheit und ist überall einsetzbar. Die Zusammensetzung erlaubt ferner die Vermeidung System-inhärenter Unterkühlungs-Effekte bzw. Erfrierungs-Effekte des behandelten Gewebes bzw. der Haut wie sie bei herkömmlichen Kältesprayformulierungen oftmals zu beobachten ist. Wie bereits umfangreich ausgeführt, ist die verlängerte Kühlwirkung insbesondere auf den Einsatz des Kälteträgermaterials zurückzuführen, wobei hierbei nochmals explizit Wasser als Kälteträgermaterial herausgestellt werden soll. Durch die Verwendung eines Bindemittels kann auch eine höhere Auftragsmenge auf die entsprechende Gewebepartie erfolgen, wodurch die Kühlintensität gesteigert werden kann.

Bei den herkömmlichen Kältesprays ist zudem ein Kälteeffekt im Wesentlichen nur für die Dauer des Aufsprühvorgangs gegeben und allenfalls für 5 bis 10 Minuten nach dem Aufsprayen. Die mit einer dermaßen kurzen Anwendungszeit nach dem Stand der Technik oftmals zu beobachtenden Rebound-Effekte, bei denen durch den Abbruch des Sprühvorgangs ein exzessives Ausdehnen der Blutgefäße bzw. eine verstärkte Schwellung erzeugt wird, können dabei ebenso verhindert werden wie eine Erhöhung des Tonus der Muskeln eines (insbesondere während eines Spiels) zu behandelnden Sportlers durch kurzfristige kräftige Kältereize. Zusammenfassend kann festgestellt werden, dass erfindungsgemäß im Regelfall eine "sanfte" Kälteeinwirkung durch Temperaturabsenkung von 5 bis 15°C über einen Zeitraum von zumindest 20 und im Regelfall zumindest 30 Minuten realisierbar ist.

Die vorstehend näher erläuterte Zusammensetzung wird nachfolgend noch anhand einer allgemeinen Beschreibung zur Herstellung der erfindungsgemäßen Zusammensetzung, mehrerer Beispiele und Figuren erläutert, ohne dass damit die generelle Aussage der vorstehenden Ausführungen eingeschränkt werden soll.

Für die erfindungsgemäße Zusammensetzung wird ein Kälteträgermaterial mit eine großen Schmelzenthalpie (beispielsweise Wasser) in einem Treibmittel mit hoher Verdampfungsenthalpie und ausgeprägter Abkühlung bei isenthalpher Druckminderung (beispielsweise Chlorethan) - emulgiert. Es bildet sich eine Wasser- Chlorethan I-Emulsion mit einer Tröpfchengröße kleiner 50 µm; Treibmittel und Kälteträgermaterial sind nicht ineinander löslich. Zur Verbesserung der Langzeitstabilität der Emulsion können Tenside hinzugegeben werden, die eine vorzeitige Koaleszenz der Wasserphase verhindern. Zur Einstellung der Auftragsmenge und damit der Kühlwirkung auf der Haut wird ein Bindemittel (beispielsweise Polyvinylacetat) verwendet.

Zur Herstellung der Zusammensetzung werden zunächst im Treibmittel Hilfsstoffe wie Tenside, Cotenside und Viskositätsmodifikatoren gelöst. Das Bindemittel wird ebenfalls vorab in der Treibgasphase vorzugsweise unter Rühren gelöst. Insbesondere wenn das Kälteträgermaterial in flüssigem Zustand vorliegt, können auch darin Hilfsstoffe wie Tenside, Cotenside und Viskositätsmodifikatoren gelöst werden. Zur Emulsionserzeugung können typische Propellerrührer/Dissolver, Rotor/Stator-Systeme oder Hochdruckhomogenisatoren Verwendung finden.

Die Zusammensetzung der vorstehend genannten Zusammensetzung aus Wasser Chlorethan und Polyvinylacetat kann wie bereits im allgemeinen Teil erläutert - je nach angestrebter Auftragsmenge, Kühlwirkung und Anwendungstemperatur - variieren. Die Substanzen können bei den spezifisch angegebenen Vertretern aber auch allgemein in der Kältesprayformulierung beispielsweise im folgenden Konzentrationsfenster vorliegen (Angaben in Gew.-%): Treibmittel 20 / Kälteträgermaterial 70 -79 / Bindemittel 1-10; Treibmittel 84-94 / Kälteträgermaterial 5/ Bindemittel 1-10.

### Beispiel 1 - Zusammensetzung 1:

In einem Becherglas werden 145 g Chlorethan bei 1-2 °C vorgelegt und bei dieser Temperatur konstant temperiert. Das Chlorethan wird mit 0,4 g Sorbitanmonooleat (Span 80, E 494, Sigma Aldrich) als Tensid versetzt. Als Bindemittel wird 10,0 g Polyvinylacetat 30 (PCAc 30 Kremers Pigmente) eingesetzt und im Chlorethan vollständig gelöst. Zur Chlorethan-Phase wird 1,0 g Menthol (racemic 98 % Sigma Aldrich) als Lokalanästhetikum hinzugegeben. Als Kälteträgermaterial wird 40 g Wasser eingesetzt, welches zur Viskositätsanpassung mit 0,6 Gummiarabikum (E414, Kremers Pigmente 63300) versetzt wird. Die wässrige Phase wird bei 10.000 U/min für 5 min mittels Rotor/Stator-Dispergierens in der Chlorethanphase emulgiert. Die fertige thermoregulierende Kältesprayformulierung kann - in Druckflaschen gefüllt - sofort eingesetzt werden. Nach längerer Lagerzeit sollte die Emulsion vor dem Einsatz kurz aufgeschüttelt werden.

Dieselbe Rezeptur wird zur Herstellung von Zusammensetzung 1' verwendet. Im Unterschied zu Zusammensetzung 1 enthält Zusammensetzung 1' allerdings kein Bindemittel.

### Beispiel 2- Zusammensetzung 2:

Die in Beispiel 1 aufgeführte Formulierung kann unter Verwendung eines Propan/Butan-Gemisches als Treibgas abgewandelt werden. Hierfür sind folgenden Änderungen nötig. In einem Druckbehälter mit Rührer/Emulgier-Vorrichtung werden 145 g Propan/Butan bei R. T. vorgelegt. Das Propan/Butan-Gemisch wird mit 0,4 g Sorbitanmonooleat (Span 80, E 494, Sigma Aldrich) als Tensid versetzt. Als Bindemittel kommt 15,0 g Cellulosenitrat, gelöst in Ethylacetat (Archäocoll 2000, Kremers Pigmente), zum Einsatz. Der Treibgasphase kann 1,0 g Menthol (racemic 98 % Sigma Aldrich) als Lokalanästhetikum hinzugegeben werden. Als PCM-Material wird 40 g Wasser eingesetzt, welches zur Viskositätsanpassung mit 0,6 Gummiarabikum (E414, Kremers Pigmente 63300) versetzt wird. Die wässrige Phase wird bei 10.000 U/min für 5 min mittels Rotor/Stator-Dispergierens in der Propan/Butan-Phase unter Druck emulgiert.

### Beispiel 3 - quantitative Untersuchung der Kühlwirkung an einem Hautmodell

Mithilfe eines Versuchsstandes wird die Kühlwirkung von Zusammensetzung 1 verglichen mit einem Kältespray aus reinem Chlorethan bewertet. Die aufgetragene Menge an reinem Chlorethan und die in Zusammensetzung 1 enthaltene Mengen an Chlorethan sind gleich groß. Die Kühlwirkung wird aus der erreichten Oberflächentemperatur und der Eindringtiefe in tiefergelegenes Muskelgewebe ermittelt. Zur Beurteilung der Kühlwirkung wurde ein Hautmodell auf Basis von Hydrogelen entwickelt welches auf eine Kerntemperatur von 37 °C und eine Oberflächentemperatur von 35 °C temperiert wird. Mittels IR-Kameras (Auflösung von 0,1K) wird der Temperaturverlauf der Oberflächen nach dem Aufbringen des Kältemittels gemessen. Über Temperatursensoren in 5 mm Tiefe im Hautmodell wird die Eindringtiefe ermittelt. Gravimetrisch wird die aufgetragene Menge bestimmen und das Abdampfverhalten des Kältesprays ermittelt.

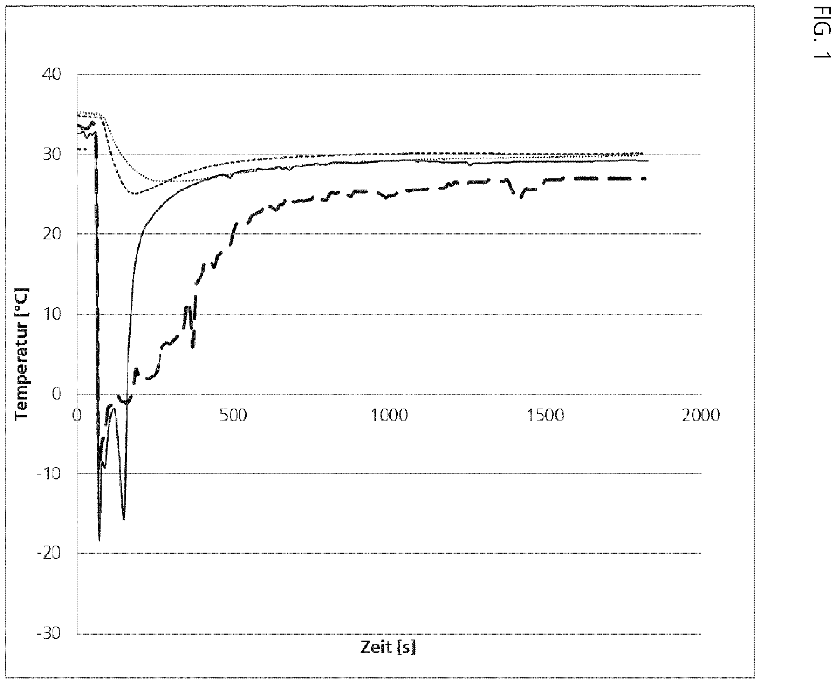

Figur 1 zeigt den Temperaturverlauf des Chlorethan-Kältesprays sowohl oberflächlich (durchgezogene Linie) als auch in 5 mm Eindringtriefe (gestrichelte Linie mit kurzen Strichen) dargestellt. Hierbei zeigt sich, dass es anfänglich zu einer oberflächlich starken Unterkühlung der Haut auf -18 °C kommt. Die Oberflächliche Temperaurabsenkung ist nach rund 5 Minuten vollständig aufgebraucht. Bei der Tiefenwirkung in 5 mm tiefe werden nach 10 min bereits wieder 30 °C erreicht. Im Vergleich dazu zeigt Formulierung 1 einen deutlich anderen Temperaturverlauf. Die Oberflächentemperatur (gestrichelte Linie mit langen Strichen) sinkt beim Aufsprühen nur bis auf -9 °C ab und verweilt länger im Bereich von 0 °C. Eine starke Unterkühlung der Haut wird verhindert und eine Schädigung der äußeren Epidermis-Schichten unterbunden. Des Weiteren wird eine Kühlwirkung auf weniger als 20 °C für etwa 10 min erreicht, auch danach verbleibt die Oberflächentemperatur für längere Zeit auf einem deutlich niedrigeren Niveau im Vergleich zum reinen Chlorethan Kältespray. Auch in der Tiefenwirkung (gepunktete Linie) wird bei Zusammensetzung 1 nicht so schnell heruntergekühlt wie bei reinem Chlorethan und über einen deutlich längeren Zeitraum eine Kältewirkung erzielt.

### Beispiel 4 - qualitative Untersuchung mit Probanden

In einer doppelt verblindeten Studie wird 40 Probanden jeweils sowohl Zusammensetzung 1 als auch Kältespray aus reinem Chlorethan auf einen Unterarm gesprüht. Die Versuche finden in einem Raum mit konstanter Raumtemperatur statt. Die Unterarme werden in Außenrotationsstellung auf den Armlehnen abgelegt und 5 cm unter der Humeroulnaren- bzw. der Humeroradialengelenklinie eine horizontale Markierungslinie gezeichnet. 15 cm distal der ersten Linie wird eine zweite gezeichnet. Die Fläche zwischen der ersten und zweiten Linie wird mit den beiden untersuchten Materialien besprüht. Die Sprays werden gleichzeitig drei Sekunden lang auf beiden Seiten gleichmäßig von distal nach proximal auf die markierte Fläche aufgesprüht. Die Probanden geben dann für beide Arme an, wenn sie das Gefühl haben, dass die Kühlwirkung auf einer Armseite nichtmehr spürbar ist. Die entsprechend gestoppte Zeit wird notiert. Zudem wird die wahrgenommene Kühlwirkung pro Seite zu Ihrem maximalen Zeitpunkt, auf einer VA·Skala von 1bis 10 ermittelt.

Alle Probanden beobachteten bei Zusammensetzung 1 eine Kühlwirkung für 25 bis 33 Minuten und beim Vergleichsspray Chlorethan eine Kühlwirung von 8 bis 11 Minuten. Die subjektiv empfundene Kühlwirkung hat sich bei der erfindungsgemäßen Zusammensetzung also verdreifacht.

### Beispiel 5 - quantitative Untersuchung der Kühlwirkung an Probanden

Auf dem Unterarm eines Probanden wird die Kühlwirkung von Zusammensetzung 1 und Zusammensetzung 1' verglichen mit einem Kältespray aus reinem Chlorethan und einem Kältespray aus einem Propan/Butan/Isobutan-Gemisch bewertet. Die aufgetragene Menge an Treibmittel ist jeweils gleich groß. Die Kühlwirkung beziehungsweise der Temperaturverlauf der Oberfläche wird mittels IR-Kameras (Auflösung von 0,1K) nach gemessen.

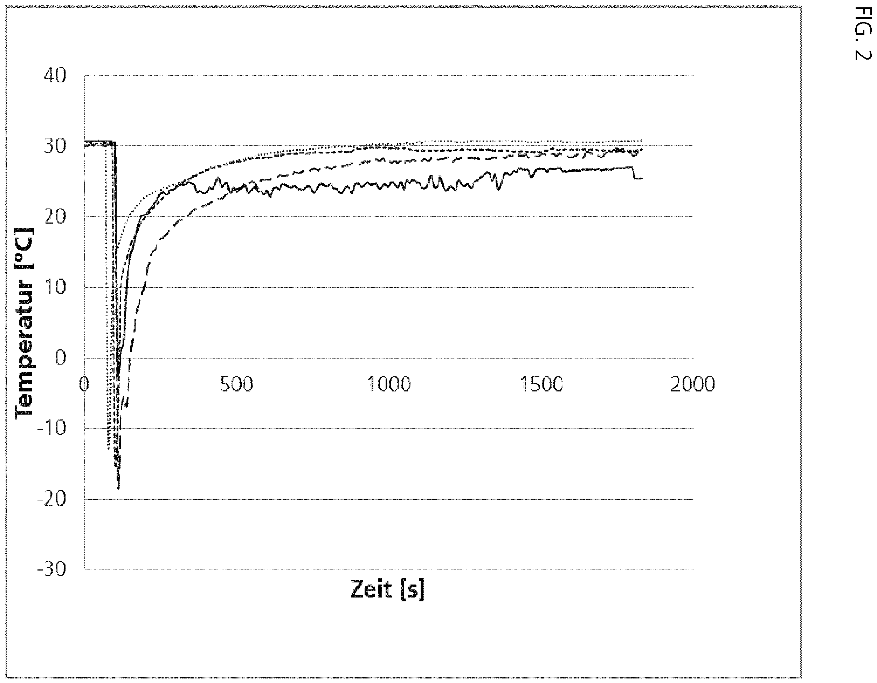

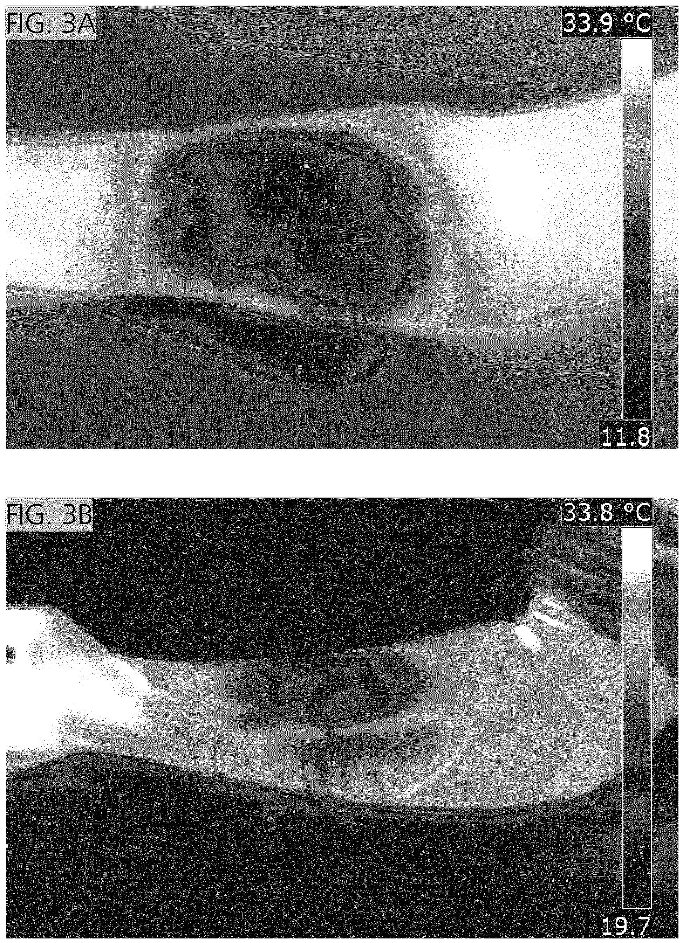

Figur 2 zeigt den Temperaturverlauf des Chlorethan-Kältesprays (gestrichelte Linie mit kurzen Strichen) und des Propan-Gemisch-Kältesprays (gepunktete Linie) verglichen mit Zusammensetzung 1 (durchgezogene Linie) und Zusammensetzung 1' (gestrichelte Linie mit langen Strichen). Während bei den herkömmlichen Kältesprays die oberflächliche Temperaurabsenkung ist nach ca. 7 Minuten nahezu vollständig aufgebraucht ist wird mit Zusammensetzung 1 und Zusammensetzung 1'eine signifikante Kühlwirkung für mindestens 25 Minuten beziehungsweise mindestens 12 Minuten erreicht. Den Unterschied zwischen den beiden zuletzt genannten Werten verdeutlichen die Figuren 3A und 3B.

Figuren 3A und 3B zeigen mittels einer IR-Kamera aufgenommene Temperaturprofile eines Unterarms der der Bindemittel-haltigen Zusammensetzung 1 (Figur 3A) und der Zusammensetzung 1' ohne Bindemittel (Figur 3B). Man erkennt deutlich in Figur 3B die auf das Auseinanderlaufen des Kälteträgermaterials zurückzuführenden Wasserstreifen, wie sie regelmäßig bei nicht ebenen zu kühlenden Gewebeflächen zu beobachten sein werden. Dagegen wird in Figur 3A das Kälteträgermaterial mittels des Bindemittels im zu kühlenden Bereich fixiert, so dass der Kühleffekt verstärkt und verlängert wird.

## Patentansprüche

1. Versprühbare Zusammensetzung zum Kühlen von Gewebe, insbesondere von menschlichem Gewebe, mittels eines schmelzenden Kälteträgermaterials, wobei das Kälteträgermaterial Wasser ist oder im Wesentlichen aus Wasser besteht, umfassend
eine Emulsion aus einem flüssigen Treibmittel mit einem Siedepunkt im Bereich von -45 °C bis +37 °C und dem in dem Treibmittel emulgierten Kälteträgermaterial.

2. Zusammensetzung nach Anspruch 1,
**dadurch gekennzeichnet, dass**
zusätzlich ein Bindemittel, insbesondere ein polymeres Bindemittel, für die präzise lokale Applikation der Zusammensetzung auf dem Gewebe enthalten ist.

3. Zusammensetzung nach einem der vorhergehenden Ansprüche,
**dadurch gekennzeichnet, dass**
das Treibmittel eine Verdampfungsenthalpie von mindestens 200 kJ/kg, insbesondere von mindestens 350 kJ/kg aufweist.

4. Zusammensetzung nach einem der vorhergehenden Ansprüche,
**dadurch gekennzeichnet, dass**
das Kälteträgermaterial eine Schmelzenthalpie von mindestens 100 kJ/kg und/oder eine Wärmeleitfähigkeit größer 0,15 W/m*K besitzt.

5. Zusammensetzung nach einem der vorhergehenden Ansprüche,
**dadurch gekennzeichnet, dass**
die mittlere in der Emulsion vorliegende Teilchengröße größer ist als 0,1 µm und insbesondere 1 bis 100 µm beträgt.

6. Zusammensetzung nach einem der zwei vorhergehenden Ansprüche,
**dadurch gekennzeichnet, dass**
das Bindemittel ein Filmbildner ist.

7. Zusammensetzung nach dem vorhergehenden Anspruch,
**dadurch gekennzeichnet, dass**
das Bindemittel im Treibmittel gelöst vorliegt.

8. Zusammensetzung nach einem der beiden vorhergehenden Ansprüche,
**dadurch gekennzeichnet, dass**
das Bindemittel eine zahlenmittlere Molmasse von 1000 bis 200000 g/mol, insbesondere von 10000 bis 100000 g/mol besitzt und eine Glastemperatur kleiner 30°C aufweist.

9. Zusammensetzung nach einem der vorhergehenden Ansprüche,
**dadurch gekennzeichnet, dass**
der Anteil des Treibmittels in der Zusammensetzung 20 bis 95 Gew.-%, insbesondere 50 bis 90 Gew.-% beträgt und der Anteil des Kälteträgermaterials in der Zusammensetzung 5 bis 80 Gew.-%, insbesondere 10 bis 50 Gew.-% beträgt.

10. Zusammensetzung nach dem vorhergehenden Anspruch,
**dadurch gekennzeichnet, dass**
in der Zusammensetzung neben dem Treibmittel und dem Kälteträgermaterial das Bindemittel in einem Anteil von bis zu 10 Gew.-% enthalten ist.

11. Zusammensetzung nach einem der beiden vorhergehenden Ansprüche,
**dadurch gekennzeichnet, dass**
bei Auftrag der Zusammensetzung auf menschliche Haut mit einer Gewebeoberflächenausgangstemperatur von 30 °C die Zeitspanne t₁, die die Dauer t der Verminderung der Gewebeoberflächenausgangstemperatur um mindestens 5 °C bezeichnet, mindestens 50% länger ist als die Zeitspanne t₂ einer entsprechenden Zusammensetzung, die kein Kälteträgermaterial enthält.

12. Zusammensetzung nach dem vorhergehenden Anspruch,
**dadurch gekennzeichnet, dass**
die Zeitspanne t₁ mindestens doppelt so groß ist wie die Zeitspanne t₂.

13. Vorrichtung zum Aufbringen einer kühlenden Zusammensetzung auf Gewebe, insbesondere auf menschlichem Gewebe mit einem ersten und einem zweiten Vorratsbehälter, einer Mischkammer und einer Sprühdüse,
wobei der erste Vorratsbehälter ein Treibmittel mit einem Siedepunkt im Bereich von -20 °C bis +37 °C und gegebenenfalls ein Bindemittel enthält und der zweite Vorratsbehälter ein Kälteträgermaterial enthält, wobei das Kälteträgermaterial Wasser ist oder im Wesentlichen aus Wasser besteht, und wobei
der erste und der zweite Behälter jeweils über ein Ventil mit einer Mischkammer verbunden sind, in der durch Vermischung des Treibmittels und des Kälteträgermaterials eine Emulsion ausgebildet wird, so dass über die Sprühdüse die Emulsion auf das zu kühlende Gewebe auftragbar ist.

## Claims

1. Sprayable composition for cooling tissue, in particular human tissue, by means of a melting coolant material, wherein the coolant material is water or is essentially composed of water, comprising an emulsion formed from a liquid propellant having a boiling point in the range from -45°C to +37°C and the coolant material emulsified in the propellant.

2. Composition according to Claim 1,
**characterized in that**
a binder, in particular a polymeric binder, is additionally present for the precise local application of the composition to the tissue.

3. Composition according to either of the preceding claims,
**characterized in that**
the propellant has an enthalpy of vaporization of at least 200 kJ/kg, in particular of at least 350 kJ/kg.

4. Composition according to any of the preceding claims,
**characterized in that**
the coolant material possesses an enthalpy of fusion of at least 100 kJ/kg and/or a thermal conductivity of greater than 0.15 W/m*K.

5. Composition according to any of the preceding claims,
**characterized in that**
the mean particle size present in the emulsion is greater than 0.1 µm and in particular is 1 to 100 µm.

6. Composition according to either of the two preceding claims,
**characterized in that**
the binder is a film former.

7. Composition according to the previous claim,
**characterized in that**
the binder is present dissolved in the propellant.

8. Composition according to either of the two preceding claims,
**characterized in that**
the binder has a number-average molar mass of from 1000 to 200 000 g/mol, in particular from 10 000 to 100 000 g/mol and has a glass transition temperature of less than 30°C.

9. Composition according to any of the preceding claims,
**characterized in that**
the proportion of the propellant in the composition is 20% to 95% by weight, in particular 50% to 90% by weight, and the proportion of the coolant material in the composition is 5% to 80% by weight, in particular 10% to 50% by weight.

10. Composition according to the preceding claim,
**characterized in that**,
in the composition, in addition to the propellant and the coolant material, the binder is present in a proportion of up to 10% by weight.

11. Composition according to either of the two preceding claims,
**characterized in that**,
when applying the composition to human skin having a tissue surface starting temperature of 30°C, the period of time t₁, which denotes the duration t of the reduction in the tissue surface starting temperature by at least 5°C, is at least 50% longer than the period of time t₂ for a corresponding composition not containing any coolant material.

12. Composition according to the preceding claim,
**characterized in that**
the period of time t₁ is at least twice as great as the period of time t₂.

13. Apparatus for applying a cooling composition to tissue, in particular to human tissue, having a first and a second storage container, a mixing chamber and a spray nozzle,
wherein the first storage container contains a propellant having a boiling point in the range from -20°C to +37°C and optionally a binder and the second storage container contains a coolant material, wherein the coolant material is water or essentially consists of water, and wherein the first and the second container are each connected to a mixing chamber via a valve, an emulsion being formed in the mixing chamber by mixing of the propellant and the coolant material so that the emulsion is applicable onto the tissue to be cooled via the spray nozzle.

## Revendications

1. Composition pulvérisable pour le refroidissement d'un tissu, notamment d'un tissu humain, au moyen d'un matériau frigoporteur fondant, le matériau frigoporteur étant de l'eau ou étant essentiellement constitué par de l'eau, comprenant
une émulsion d'un agent gonflant liquide ayant un point d'ébullition dans la plage allant de -45 °C à +37 °C et le matériau frigoporteur émulsifié dans l'agent gonflant.

2. Composition selon la revendication 1, **caractérisée en ce qu'**un liant, notamment un liant polymère, est en outre contenu pour l'application locale précise de la composition sur le tissu.

3. Composition selon l'une quelconque des revendications précédentes, **caractérisée en ce que** l'agent gonflant présente une enthalpie d'évaporation d'au moins 200 kJ/kg, notamment d'au moins 350 kJ/kg.

4. Composition selon l'une quelconque des revendications précédentes, **caractérisée en ce que** le matériau frigoporteur présente une enthalpie de fusion d'au moins 100 kJ/kg et/ou une conductivité thermique supérieure à 0,15 W/m*K.

5. Composition selon l'une quelconque des revendications précédentes, **caractérisée en ce que** la taille de particule moyenne présente dans l'émulsion est supérieure à 0,1 µm et notamment est de 1 à 100 µm.

6. Composition selon l'une quelconque des deux revendications précédentes, **caractérisée en ce que** le liant est un agent filmogène.

7. Composition selon la revendication précédente, **caractérisée en ce que** le liant est présent sous forme dissoute dans l'agent gonflant.

8. Composition selon l'une quelconque des deux revendications précédentes, **caractérisée en ce que** le liant présente une masse molaire moyenne en nombre de 1000 à 200 000 g/mol, notamment de 10 000 à 100 000 g/mol, et une température de transition vitreuse inférieure à 30 °C.

9. Composition selon l'une quelconque des revendications précédentes, **caractérisée en ce que** la proportion de l'agent gonflant dans la composition est de 20 à 95 % en poids, notamment de 50 à 90 % en poids, et la proportion du matériau frigoporteur dans la composition est de 5 à 80 % en poids, notamment de 10 à 50 % en poids.

10. Composition selon la revendication précédente, **caractérisée en ce qu'**outre l'agent gonflant et le matériau frigoporteur, le liant est contenu dans la composition en une proportion de jusqu'à 10 % en poids.

11. Composition selon l'une quelconque des deux revendications précédentes, **caractérisée en ce que**, lors de l'application de la composition sur la peau humaine ayant une température initiale de surface de tissu de 30 °C, la période de temps t₁, qui désigne la durée t de la réduction de la température initiale de surface de tissu d'au moins 5 °C, est au moins 50 % plus longue que la période de temps t₂ d'une composition correspondante qui ne contient pas de matériau frigoporteur.

12. Composition selon la revendication précédente, **caractérisée en ce que** la période de temps t₁ est au moins deux fois plus grande que la période de temps t₂.

13. Dispositif pour l'application d'une composition refroidissante sur un tissu, notamment sur un tissu humain, pourvu d'un premier et d'un deuxième contenant de stockage, d'une chambre de mélange et d'une buse de pulvérisation,
dans lequel le premier contenant de stockage contient un agent gonflant ayant un point d'ébullition dans la plage allant de -20 °C à +37 °C et éventuellement un liant, et le deuxième contenant de stockage contient un matériau frigoporteur, le matériau frigoporteur étant de l'eau ou étant essentiellement constitué par de l'eau, et dans lequel
le premier et le deuxième contenant sont chacun reliés par l'intermédiaire d'une soupape avec une chambre de mélange, dans laquelle une émulsion est formée par mélange de l'agent gonflant et du matériau frigoporteur, de telle sorte que l'émulsion puisse être appliquée sur le tissu à refroidir par l'intermédiaire de la buse de pulvérisation.
